# EUROPEAN PATENT APPLICATION

(11) **EP 3 598 934 A1**
(43) Date of publication of application: **29.01.2020**
(21) Application number: 19155368.4
(22) Date of filing: 04.02.2019
(51) Int. Cl.: A61B 5/00, A61B 5/024, A61B 5/1455

(54) **BIOMETRIC INFORMATION DETECTION DEVICE AND METHOD OF PRODUCING THE SAME, AND BIOMETRIC INFORMATION DETECTION MODULE AND METHOD OF PRODUCING THE SAME**

(30) Priority: 27.07.2018 JP 2018141420
(71) Applicant: Seiko Instruments Inc., Chiba-shi, Chiba 261-8507 (JP); Showa University, Tokyo 142-8555 (JP)
(72) Inventor: YOSHIDA, Yoshifumi, Chiba-shi, Chiba 261-8507 (JP); MAKI, Kotaro, Tokyo, Tokyo 142-8555 (JP)
(74) Representative: Miller Sturt Kenyon

(57) **Abstract**

This biometric information detection device includes: a sensor including a light emitting element that is able to emit light and a light receiving element that is able to receive the light emitted from the light emitting element; a sealing member which includes a first light-transmitting part that covers the light emitting element and is able to transmit the light and a second light-transmitting part that covers the light receiving element and is able to transmit the light, and seals the sensor; and a holding member that is attached to at least one of the teeth and the gums and is able to hold the sealing member.

## Description

### BACKGROUND OF THE INVENTION

### Field of the Invention

The present disclosure relates to a biometric information detection module configured to detect biometric information of a subject, a biometric information detection device including the same, and method of producing the same.

### Description of Related Art

Several biological monitors that are installed in the oral cavity to obtain biometric information have been proposed so far (for example, refer to Japanese Unexamined Patent Application, First Publication No. 2004-167120, and United States Patent No. 8771149).

### SUMMARY OF THE INVENTION

In such devices, it is desirable to secure high safety and maintain an accurate operation for a long time.

Therefore, it is desirable to provide a biometric information detection module and a biometric information detection device having excellent safety and long-term reliability and a method of producing the same.

A biometric information detection device according to one embodiment of the present disclosure is installed in the oral cavity and includes a sensor, a sealing member, and a holding member. The sensor includes a light emitting element that can emit light and a light receiving element that can receive light emitted from the light emitting element. The sealing member includes a first light-transmitting part that covers the light emitting element and can transmit light and a second light-transmitting part that covers the light receiving element and can transmit light, and seals the sensor. The holding member is attached to at least one of the teeth and the gums and can hold the sealing member.

According to a biometric information detection device of one embodiment of the present disclosure, since a sensor is sealed with a sealing member, both high waterproofness with respect to the sensor and high safety for a living body are secured, and excellent long-term reliability is obtained.

### BRIEF DESCRIPTION OF THE DRAWINGS

FIG. 1 is a perspective view showing an appearance of a biometric information detection device according to one embodiment of the present disclosure.
FIG. 2 is a cross-sectional view showing a schematic cross-sectional configuration example of the biometric information detection device shown in FIG. 1.
FIG. 3 is a perspective view showing an example in which the biometric information detection device shown in FIG 1 is installed in the oral cavity.
FIG. 4 is a block diagram showing an overall configuration example of the biometric information detection device shown in FIG. 1.
FIG. 5 is a characteristic diagram showing light receiving wavelength ranges of devices that can be applied to a light receiving element of a light sensor shown in FIG. 1.
FIG. 6A is an explanatory diagram showing one process of a method of producing the biometric information detection device shown in FIG. 1.
FIG. 6B is an explanatory diagram showing one process following FIG. 6A.
FIG. 7 is a cross-sectional view showing a schematic configuration example of a biometric information detection device as Modified Example 1 of the present disclosure.
FIG. 8 is a cross-sectional view showing a schematic configuration example of a biometric information detection device as Modified Example 2 of the present disclosure.
FIG. 9 is a perspective view showing a schematic configuration example of a biometric information detection device as Modified Example 3 of the present disclosure.

### DETAILED DESCRIPTION OF THE INVENTION

Embodiments of the present disclosure will be described below in detail with reference to the drawings. Here, the description will be made in the following order.
1. Embodiment (example of mouthpiece-like biometric information detection device in which a light sensor that receives reflected light from the gums is sealed)
2. Modified examples
   Modified Example 1 (example of biometric information detection device that receives reflected light from parts other than the gums)
   Modified Example 2 (example of biometric information detection device including strain sensor)
   Modified Example 3 (example of biometric information detection device including acceleration sensor)
3. Other modified examples

### <1. Embodiment>

### [Overall configuration of biometric information detection device 1]

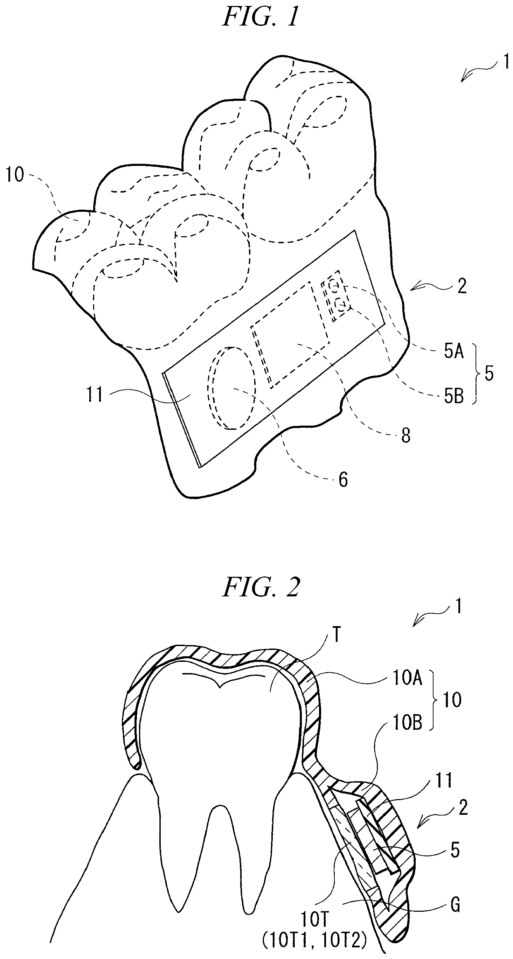

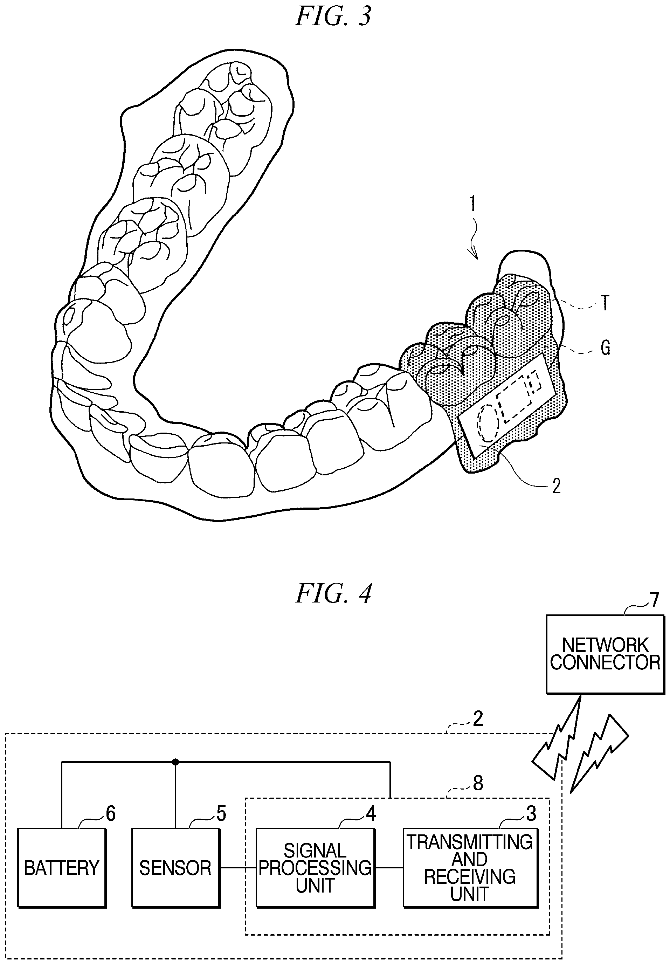

FIG. 1 is a perspective view schematically showing a schematic configuration example of a biometric information detection device 1 according to one embodiment of the present disclosure. In addition, FIG. 2 is a cross-sectional view schematically showing a schematic configuration example of the biometric information detection device 1. In addition, FIG. 3 is a perspective view showing an example of a use state of the biometric information detection device 1. Further, FIG. 4 is a block diagram showing a configuration of a biometric information detection module 2 included in the biometric information detection device 1.

As shown in FIG. 3, the biometric information detection device 1 is installed in the oral cavity and is installed to cover, for example, some teeth T and a part of the gums G.

As shown in FIGS. 1 and 2, the biometric information detection device 1 includes a shell 10 covering the teeth T and the gums G, and the biometric information detection module 2 built into the shell 10.

The shell 10 has a mouthpiece shape in which an upper part 10A covering the teeth T and a lower part 10B covering the gums G are integrated, and is made of, for example, a thermoplastic polymer compound. As the thermoplastic polymer compound, for example, compounds including polyethylene, polyurethane, acrylic or the like, can be applied. The biometric information detection module 2 is embedded in the lower part 10B so that it is sealed from the outside. Therefore, the lower part 10B of the shell 10 is one specific example corresponding to a "sealing member" of the present disclosure. In this manner, since the biometric information detection module 2 is embedded in the lower part 10B of the shell 10, it is reliably guarded from saliva in the oral cavity, water of drink and the like, and outside air. In addition, since the upper part 10A of the shell 10 is integrated with the lower part 10B, it corresponds to one specific example of a "holding member" of the present disclosure which holds the lower part 10B as a sealing member. Here, for the convenience of explanation, FIG. 1 shows a state in which a part of the shell 10 covering the biometric information detection module 2 is removed in order to improve the visibility. However, actually, since the biometric information detection module 2 is covered with the shell 10, it is not visible from the outside unless the shell 10 is transparent.

In the biometric information detection module 2, for example, a battery 6, a transmitting and receiving module 8, and a light sensor 5 are disposed on a mounting board 11 (refer to FIGS. 1, 2, and 4).

The mounting board 11 is, for example, a sheet-like flexible board on which a wiring is printed.

The battery 6 functions as a power supply configured to supply power for driving the light sensor 5 and the transmitting and receiving module 8 to the light sensor 5 and the transmitting and receiving module 8. The battery 6 is, for example, a button type or coin type primary battery. Alternatively, as the battery 6, a secondary battery may be applied as long as it can perform non-contact charging. In addition, the structure of the battery 6 is not particularly limited, and may be, for example, a battery having an exterior in which a metal foil and a laminate film are bonded and having excellent flexibility.

As shown in FIG. 4, the transmitting and receiving module 8 includes, for example a transmitting and receiving unit 3 and a signal processing unit 4. Generally, chips in which the transmitting and receiving unit 3 and the signal processing unit 4 are integrated are distributed. The transmitting and receiving unit 3 is an antenna that performs wireless type data communication with a network connector 7 provided outside the biometric information detection module 2. Here, the network connector 7 is a communication device, for example, a personal computer, a tablet terminal, or a smartphone, that can be connected to a network such as the Internet. In addition, the signal processing unit 4 is, for example, a signal processing circuit including a memory, a microprocessor, an analog-to-digital (A/D) converter, and the like. A detection signal (output signal) from the light sensor 5 is input to the signal processing unit 4. The signal processing unit 4 receives supply of power from the battery 6 and is driven, and generates a digital data signal that can be transmitted through the transmitting and receiving unit 3 based on the output signal from the light sensor 5. The transmitting and receiving unit 3 receives supply of power from the battery 6 and is driven, and can transmit wirelessly the digital data signal from the signal processing unit 4 to an external network through the network connector 7. In addition, the transmitting and receiving unit 3 can receive wirelessly a control signal from the external network through the network connector 7. Here, in FIG. 1 and the like, the mounting board 11 has a rectangular planar shape, and the battery 6, the transmitting and receiving module 8, and the light sensor 5 are disposed in a row. However, the present disclosure is not limited thereto, and the layout of the battery 6, the transmitting and receiving module 8 and the light sensor 5 can be arbitrarily set.

The light sensor 5 is, for example, a reflective photoelectric sensor, and includes a light emitting element 5A that can emit light such as a light emitting diode and a light receiving element 5B that can receive light emitted from the light emitting element 5A such as a photodiode. Light emitted from the light emitting element 5A has, for example, a wavelength of 400 nm or more and 1,000 nm or less. A part of the lower part 10B of the shell 10 covering the biometric information detection module 2, which covers the light emitting element 5A, is a first light-transmitting part 10T1 that can transmit light emitted from the light emitting element 5A (refer to FIG. 2). In addition, a part of the lower part 10B of the shell 10, which covers the light receiving element 5B, is a second light-transmitting part 10T2 that can transmit light that is reflected from the inside of the gums G or the vicinity of the surface of the gums G from light emitted from the light emitting element 5A (refer to FIG. 2). The first light-transmitting part 10T1 and the second light-transmitting part 10T2 may also be made of a thermoplastic polymer compound. Here, a constituent material of the first light-transmitting part 10T1 and a constituent material of the second light-transmitting part 10T2 may be substantially the same material, and, they may be constituted using, for example, a transparent material among the above-described thermoplastic polymer compounds. In addition, the first light-transmitting part 10T1 and the second light-transmitting part 10T2 need not be separated, and may be continuously formed. This is advantageous for integrated formation. In addition, the entire lower part 10B may be transparent, and the entire shell 10 in which the upper part 10A is incorporated into the lower part 10B may be transparent. That is, in the shell 10, the first light-transmitting part 10T1 covering the light emitting element 5A and the second light-transmitting part 10T2 covering the light receiving element 5B (these are collectively referred to as the light-transmitting part 10T) may have light transmitting properties, and parts other than the light-transmitting part 10T may have light shielding properties or light transmitting properties.

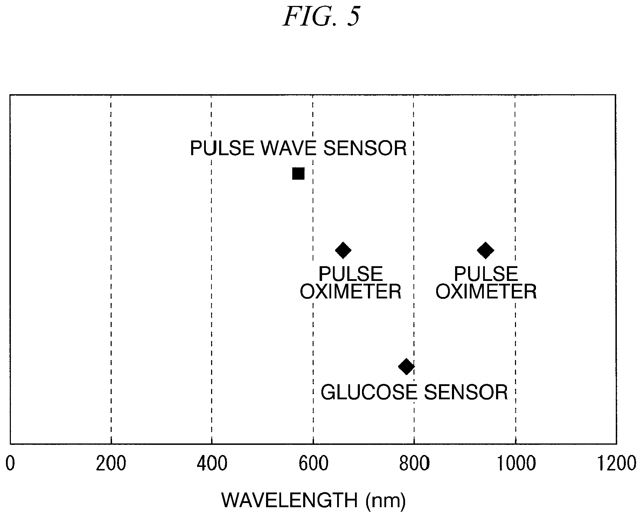

The light emitting element 5A is disposed to face the gums G with the first light-transmitting part 10T1 therebetween. The light receiving element 5B is disposed near the light emitting element 5A so that it faces the gums G with the second light-transmitting part 10T2 therebetween. Therefore, light emitted from the light emitting element 5A passes through the first light-transmitting part 10T1 of the shell 10, reaches the gums G, and is reflected at the gums G. Light reflected from the gums G passes through the second light-transmitting part 10T2 of the shell 10 and enters the light receiving element 5B. Reflected light incident on the light receiving element 5B includes, for example, biometric information such as a blood flow rate. The light sensor 5 is, for example, a pulse wave sensor configured to detect a pulse wave using the above biometric information, a pulse oximeter configured to detect percutaneous arterial oxygen saturation (SpO₂) and a pulse rate using the above biometric information, or a glucose sensor configured to detect a glucose concentration using the above biometric information. Here, the light receiving element 5B of the light sensor 5 has different light receiving wavelength ranges according to types of biometric information to be acquired. FIG 5 shows light receiving wavelength ranges in devices suitable for acquiring various types of biometric information. As shown in FIG. 5, the light receiving element 5B that can receive light with a wavelength of about 570 nm is used for the pulse wave sensor, the light receiving element 5B that can receive light with a wavelength of about 600 nm to 1,000 nm is used for the pulse oximeter configured to measure percutaneous arterial oxygen saturation, and the light receiving element 5B that can receive light with a wavelength of about 750 nm is used for the glucose sensor configured to detect a glucose concentration.

### [Method of producing the biometric information detection device 1]

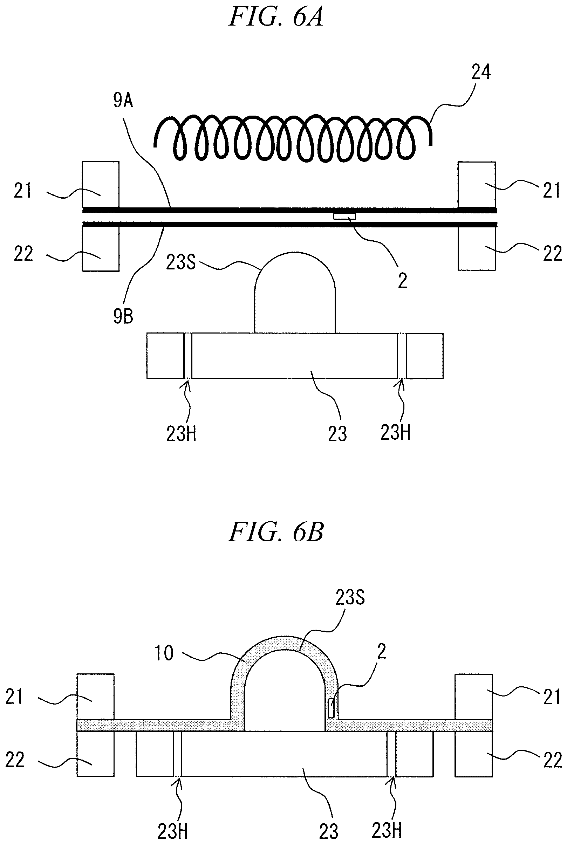

Next, a method of producing the biometric information detection device 1 will be described with reference to FIG. 6A and FIG. 6B in addition to FIG. 1 to FIG. 4. FIG. 6A is an explanatory diagram showing one process of the method of producing the biometric information detection device 1 and FIG. 6B is an explanatory diagram showing one process following FIG. 6A. Here, FIG. 6A and FIG. 6B are schematic diagrams showing only main members related to the explanation of this production process.

In order to produce the biometric information detection device 1, first, the above biometric information detection module 2 is prepared. Next, for example, as shown in FIG. 6A, the biometric information detection module 2 is placed between two sealing films 9A and 9B made of a thermoplastic polymer compound. Here, the sealing film 9A is held by a holding member 21 and the sealing film 9B is held by the holding member 21. Meanwhile, a tooth form 23 matching the teeth T and the gums G of a user who will wear the biometric information detection device 1 is prepared. The tooth form 23 can be molded by, for example, stamping or a 3D printer. Then, a part of the sealing films 9A and 9B in the vicinity of the light sensor 5 is heated to a temperature of about 280 °C by a heater 24, and as shown in FIG 6B, the tooth form 23 is pressed against the sealing films 9A and 9B and subjected to molding. Here, a heating temperature of the heater 24 is not limited to the above 280 °C, and it can be arbitrarily set, for example, within a range of 100 °C to 300 °C. During molding, when air in the gap between the tooth form 23 and the sealing films 9A and 9B is discharged from a through-hole 23H of the tooth form 23, a surface 23S of the tooth form 23 and the sealing films 9A and 9B are brought into close contact with each other. Therefore, the sealing films 9A and 9B are deformed into a shape conforming to the surface 23S of the tooth form 23 and welded. Then, when the tooth form 23 and the welded sealing films 9A and 9B are cooled, the mouthpiece-like shell 10 is obtained. When the shell 10 is separated from the tooth form 23, the biometric information detection device 1 is obtained.

Here, in the present embodiment, the upper part 10A as a holding member and the lower part 10B as a sealing member are integrally formed by molding using the tooth form 23. However, for example, only the lower part 10B is produced according to the above vacuum forming, and, for example, a holding member such as a metal fitting that engages with the teeth T may be bonded to the lower part 10B. In addition, in FIG. 6A and FIG. 6B, the tooth form 23 having the surface 23S with a very simplified shape is shown. However, actually, the surface 23S of the tooth form 23 has a unique shape according to each user.

### [Operations and effects]

In this manner, according to the biometric information detection module 2 and the biometric information detection device 1 of the present embodiment, the light sensor 5 acquires an output signal including biometric information, and the output signal is transmitted from the transmitting and receiving unit 3 to the network connector 7 through the signal processing unit 4, and can be additionally transmitted to an external network. When this output signal is analyzed, it is possible to obtain specific biometric information due to a disease of a patient who is a user in whom the biometric information detection device 1 is installed. In addition, it is possible to recognize change in daily health conditions in healthy users and maintain and manage health conditions. These information items can be very useful information for users themselves or doctors in charge thereof.

In the related art, for example, as disclosed in the above Patent Document 1 and Patent Document 2, attempts to detect biometric information by installing a sensor in the oral cavity have been made. However, a part of a sensor in a sensor module or the entire sensor, or some of other constituent members in a sensor module or all of the other constituent members are exposed in the oral cavity. Thus, there is a concern of an influence on a living body and it is thought that the deterioration of the sensor and deterioration of the sensor module are likely to occur. On the other hand, according to the biometric information detection module 2 and the biometric information detection device 1, since the light sensor 5 is sealed with the shell 10 as a sealing member, high waterproofness and high safety are secured. Therefore, the biometric information detection module 2 and the biometric information detection device 1 have superior long-term reliability to that of a biometric information detection module of the related art.

In addition, in the biometric information detection module 2 and the biometric information detection device 1, in the lower part 10B of the shell 10, the first light-transmitting part 10T1 and the second light-transmitting part 10T2 are provided and emit light outside of the lower part 10B of the shell 10 and receive light from the outside. Therefore, the light sensor 5 can obtain biometric information from, for example, the teeth T and the gums G with high accuracy even if it is sealed with the shell 10. On the other hand, it is thought that, in a sensor module of the related art, since it is necessary to expose a sensor in the oral cavity, deterioration such as corrosion of the sensor itself is likely to occur. In the sensor module of the related art, even if only a terminal part of a sensor is exposed in the oral cavity and the other part is molded with a resin or the like, this is insufficient in consideration of long-term reliability because a possibility of water entering from an interface between the exposed part and the molded part still remains.

Since the light sensor 5 is held by the upper part 10A via the lower part 10B, relative positions between the light sensor 5, and the teeth T and the gums G are stable, and improvement in detection accuracy can be expected.

In addition, in the light sensor 5, light is emitted to the gums G from the light emitting element 5A disposed to face the gums G, and light reflected from the gums G is detected by the light receiving element 5B disposed to face the gums G. Therefore, for example, compared with the case in which light is emitted to the skin outside the oral cavity and reflected light is detected, biometric information with higher accuracy is obtained. In addition, since the biometric information detection module 2 is always disposed in the oral cavity in a relatively stable temperature environment, biometric information with higher accuracy is obtained compared with the case in which the biometric information detection module 2 is attached outside the oral cavity, for example, to the arms and fingers.

### <2. Modified Examples>

Next, modified examples (Modified Examples 1 to 3) of the above embodiment will be described. Here, components the same as those in the embodiment will be denoted with the same reference numerals and descriptions thereof will be appropriately omitted.

### [Modified Example 1]

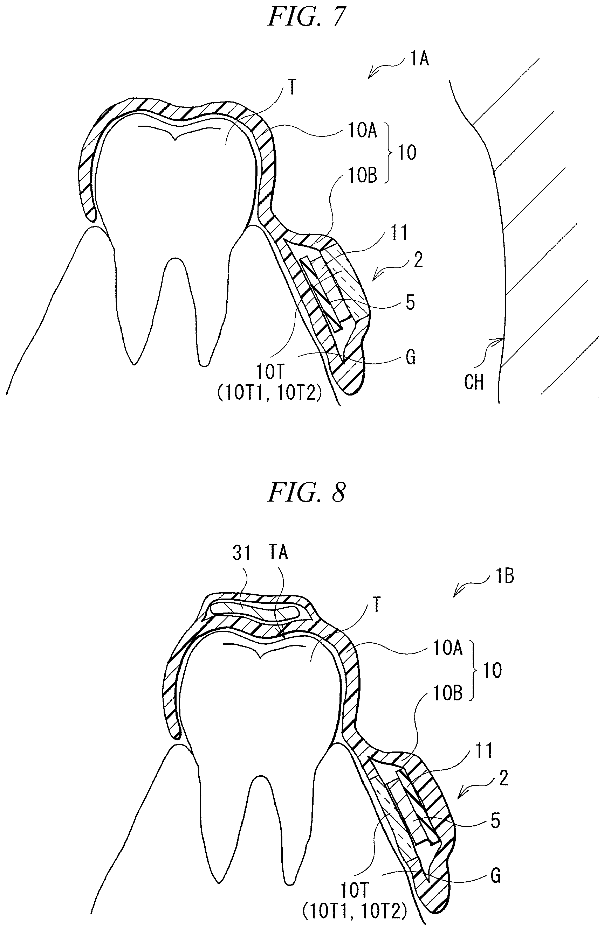

The biometric information detection device 1 of the above embodiment emits light to the gums G, receives light reflected from the gums G, and thus detects biometric information. On the other hand, a biometric information detection device 1A of Modified Example 1 of the present disclosure shown in FIG. 7 emits light to an inner surface of a cheek CH in the oral cavity, receives light reflected from the inner surface of the cheek CH, and thus detects biometric information. In the biometric information detection device 1A, the light emitting element 5A is disposed to face the cheek CH with the first light-transmitting part 10T1 therebetween, and the light receiving element 5B is disposed near the light emitting element 5A so that it faces the cheek CH with the second light-transmitting part 10T2 therebetween. Therefore, the light-transmitting part 10T of the lower part 10B of the shell 10 covering the biometric information detection module 2 is provided on the side opposite to the gums G with the biometric information detection module 2 therebetween. Except for these points, the biometric information detection device 1A has substantially the same configuration as the biometric information detection device 1 of the above embodiment. Also in the biometric information detection device 1A, the same effects as in the biometric information detection device 1 of the above embodiment are obtained. For example, the biometric information detection device 1A of this modified example is beneficial when it is determined that a surface structure of the gums G of a subject is not favorable and it is difficult to obtain stable biometric information.

### [Modified Example 2]

FIG. 8 is a cross-sectional view showing a schematic configuration of a biometric information detection device 1B as Modified Example 2 of the present disclosure. In the biometric information detection device 1B, a strain sensor 31 is embedded in a part of the shell 10 covering a crown part TA of the teeth T. The strain sensor 31 is connected to the battery 6 through a power line and is connected to the signal processing unit 4 through a communication line. The strain sensor 31 receives supply of power from the battery 6 and transmits a detection signal to the signal processing unit 4. Except for these points, the biometric information detection device 1B has substantially the same configuration as the biometric information detection device 1 of the above embodiment. Therefore, also in the biometric information detection device 1B, the same effects as in the biometric information detection device 1 of the above embodiment are obtained. Moreover, when the strain sensor 31 is provided, it is possible to measure the biting strength when the teeth of the upper jaw and the teeth of the lower jaw of a subject are engaged and the pressure of the cheek or tongue of a subject in contact with the strain sensor 31. When information such as the biting strength and the pressure of the cheek and tongue is used, it is possible to obtain structural strength information and teeth alignment information necessary for artificial teeth, for example, dentures and implants. Here, as the strain sensor 31, for example, a magnetostriction sensor using magnetostriction can be applied. In addition, a pressure sensor using a piezoelectric element or the like can be applied in place of the strain sensor 31.

### [Modified Example 3]

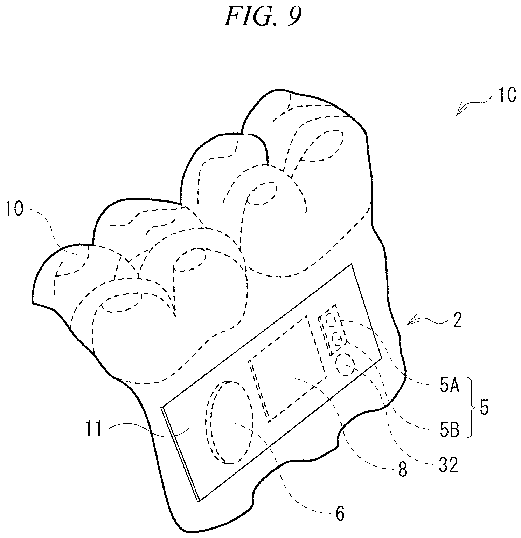

FIG. 9 is a perspective view showing a schematic configuration of a biometric information detection device 1C as Modified Example 3 of the present disclosure. In the biometric information detection device 1C, for example, an acceleration sensor 32 is additionally provided in the biometric information detection module 2. The acceleration sensor 32 is connected to the battery 6 through a power line and is connected to the signal processing unit 4 through a communication line. The acceleration sensor 32 receives supply of power from the battery 6 and transmits a detection signal to the signal processing unit 4. Except for these points, the biometric information detection device 1C has substantially the same configuration as the biometric information detection device 1 of the above embodiment. Therefore, also in the biometric information detection device 1C, the same effects as in the biometric information detection device 1 of the above embodiment are obtained. Then, when the acceleration sensor 32 is provided, for example, it is possible to detect a movement of the lower jaw with respect to the upper jaw of the subject. When a relative movement between the upper jaw and the lower jaw is detected, for example, it is possible to obtain structural strength information necessary for artificial teeth such as dentures and implants and this may be used for diagnosis of the presence of jaw joint abnormalities. In addition, a gyro sensor may be used in place of the acceleration sensor 32.

### <3. Other modified examples>

While the present disclosure has been described above with reference to the embodiment and several modified examples, the present disclosure is not limited to the embodiment and the like, and various modifications can be made.

For example, while the biometric information detection device 1 having the shell 10 mounted so that it covers two consecutive crown parts has been exemplified in the above embodiment and the like, the biometric information detection device of the present disclosure is not limited thereto. The biometric information detection device of the present disclosure may have, for example, a shell mounted so that it covers only one crown part or a shell mounted so that it covers three or more crown parts. However, in consideration of preventing accidental ingestion, it is desirable to have a shell as large as possible. For example, it is more desirable to have a shell with a size that covers all teeth of the lower jaw or all teeth of the upper jaw.

In addition, while the biometric information detection device including one light sensor has been described in the above embodiment, the biometric information detection device of the present disclosure may include two or more light sensors. In addition, the biometric information detection device may be mounted on both the upper jaw and the lower jaw.

Here, effects described in this specification are only examples, and are not limited, and other effects may be provided.

In addition, the present disclosure can have the following configurations.
(1) A biometric information detection device installed in the oral cavity, including:
   a sensor including a light emitting element that is able to emit light and a light receiving element that is able to receive the light emitted from the light emitting element;
   a sealing member which includes a first light-transmitting part that covers the light emitting element and is able to transmit the light and a second light-transmitting part that covers the light receiving element and is able to transmit the light, and seals the sensor; and
   a holding member that is attached to at least one of the teeth and the gums and is able to hold the sealing member.
(2) The biometric information detection device according to (1),
   wherein the light emitting element is disposed to face the gums with the first light-transmitting part therebetween, and
   wherein the light receiving element is disposed to face the gums with the second light-transmitting part therebetween.
(3) The biometric information detection device according to (1),
   wherein the light emitting element is disposed to face an inner surface of the cheek with the first light-transmitting part therebetween, and
   wherein the light receiving element is disposed to face an inner surface of the cheek with the second light-transmitting part therebetween.
(4) The biometric information detection device according to any one of (1) to (3),
   wherein the light has a wavelength of 400 nm or more and 1,000 nm or less.
(5) The biometric information detection device according to any one of (1) to (4), further including
   a battery that is sealed with the sealing member and supplies power to the sensor.
(6) The biometric information detection device according to (5), further including
   a signal processing unit that is sealed with the sealing member, and receives supply of power from the battery and generates a data signal based on an output signal from the sensor, and
   a transmitting and receiving unit that is sealed with the sealing member and receives supply of power from the battery, is able to transmit the data signal from the signal processing unit to an external device and is able to receive a control signal from the external device.
(7) The biometric information detection device according to any one of (1) to (6),
   wherein the first light-transmitting part and the second light-transmitting part are made of a thermoplastic polymer compound.
(8) The biometric information detection device according to any one of (1) to (7),
   wherein the sensor is a pulse wave sensor, a pulse oximeter or a glucose sensor.
(9) The biometric information detection device according to any one of (1) to (8),
   wherein the holding member and the sealing member are an integrally formed mouthpiece.
(10) The biometric information detection device according to any one of (1) to (9), further including
   another sensor configured to measure a physical quantity.
(11) A biometric information detection module including:
   a sensor including a light emitting element that is able to emit light and a light receiving element that is able to receive the light emitted from the light emitting element; and
   a sealing member which includes a first light-transmitting part that covers the light emitting element and is able to transmit the light and a second light-transmitting part that covers the light receiving element and is able to transmit the light, and seals the sensor.
(12) A method of producing a biometric information detection device installed in the oral cavity, including:
   preparing a sensor including a light emitting element that is able to emit light and a light receiving element that is able to receive the light emitted from the light emitting element;
   inserting the sensor between a pair of sealing films, heating and molding the pair of sealing films, and thereby forming a sealing member with which the sensor is sealed; and
   forming a holding member that is attached to at least one of the teeth and the gums and holds the sealing member.
(13) The method of producing a biometric information detection device according to (12),
   wherein the sealing member and the holding member are integrally formed by the molding.
(14) A method of producing a biometric information detection module including:
   preparing a sensor including a light emitting element that is able to emit light and a light receiving element that is able to receive the light emitted from the light emitting element; and
   inserting the sensor between a pair of sealing films, heating and molding the pair of sealing films, and thereby forming a sealing member with which the sensor is sealed.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

### EXPLANATION OF REFERENCES

1, 1A to 1C Biometric information detection device
2 Biometric information detection module
3 Transmitting and receiving unit
4 Signal processing unit
5 Light sensor
6 Battery
7 Network connector
8 Transmitting and receiving module
9A, 9B Sealing film
10 Shell
11 Mounting board
23 Tooth form
24 Heater
31 Strain sensor
32 Acceleration sensor
G Gums
T Teeth

## Claims

1. A biometric information detection device installed in the oral cavity, comprising:
a sensor including a light emitting element that is able to emit light and a light receiving element that is able to receive the light emitted from the light emitting element;
a sealing member which includes a first light-transmitting part that covers the light emitting element and is able to transmit the light and a second light-transmitting part that covers the light receiving element and is able to transmit the light, and seals the sensor; and
a holding member that is attached to at least one of the teeth and the gums and is able to hold the sealing member.

2. The biometric information detection device according to claim 1,
wherein the light emitting element is disposed to face the gums with the first light-transmitting part therebetween, and
wherein the light receiving element is disposed to face the gums with the second light-transmitting part therebetween.

3. The biometric information detection device according to claim 1,
wherein the light emitting element is disposed to face an inner surface of the cheek with the first light-transmitting part therebetween, and
wherein the light receiving element is disposed to face an inner surface of the cheek with the second light-transmitting part therebetween.

4. The biometric information detection device according to any one of claims 1 to 3, wherein the light has a wavelength of 400 nm or more and 1,000 nm or less.

5. The biometric information detection device according to any one of claims 1 to 4 further comprising
a battery that is sealed with the sealing member and supplies power to the sensor.

6. The biometric information detection device according to claim 5, further comprising
a signal processing unit that is sealed with the sealing member, and receives supply of power from the battery and generates a data signal based on an output signal from the sensor, and
a transmitting and receiving unit that is sealed with the sealing member and receives supply of power from the battery, is able to transmit the data signal from the signal processing unit to an external device and is able to receive a control signal from the external device.

7. The biometric information detection device according to any one of claims 1 to 6
wherein the first light-transmitting part and the second light-transmitting part are made of a thermoplastic polymer compound.

8. The biometric information detection device according to any one of claims 1 to 7,
wherein the sensor is a pulse wave sensor, a pulse oximeter or a glucose sensor.

9. The biometric information detection device according to any one of claims 1 to 8,
wherein the holding member and the sealing member are an integrally formed mouthpiece.

10. The biometric information detection device according to any one of claims 1 to 9, further comprising
another sensor configured to measure a physical quantity.

11. A biometric information detection module comprising:
a sensor including a light emitting element that is able to emit light and a light receiving element that is able to receive the light emitted from the light emitting element; and
a sealing member which includes a first light-transmitting part that covers the light emitting element and is able to transmit the light and a second light-transmitting part that covers the light receiving element and is able to transmit the light, and seals the sensor.

12. A method of producing a biometric information detection device installed in the oral cavity, comprising:
preparing a sensor including a light emitting element that is able to emit light and a light receiving element that is able to receive the light emitted from the light emitting element;
after inserting the sensor between a pair of sealing films, heating and molding the pair of sealing films, and thereby forming a sealing member with which the sensor is sealed; and
forming a holding member that is attached to at least one of the teeth and the gums and holds the sealing member.

13. The method of producing a biometric information detection device according to claim 12,
wherein the sealing member and the holding member are integrally formed by the molding.

14. A method of producing a biometric information detection module comprising:
preparing a sensor including a light emitting element that is able to emit light and a light receiving element that is able to receive the light emitted from the light emitting element; and
after inserting the sensor between a pair of sealing films, heating and molding the pair of sealing films, and thereby forming a sealing member with which the sensor is sealed.
